# EUROPEAN PATENT SPECIFICATION

(11) **EP 1 196 033 B1**
(45) Date of publication and mention of the grant of the patent: **01.12.2004**
(21) Application number: 00937966.0
(22) Date of filing: 01.06.2000
(51) Int. Cl.: A01N 59/00

(54) **A COMPOSITION AND METHOD FOR CLEANING SURFACES**
ZUSAMMENSETZUNG UND VERFAHREN ZUR REINIGUNG VON FLÄCHEN
COMPOSITION ET PROCEDE POUR LE NETTOYAGE DE SURFACES

(30) Priority: 02.06.1999 US 324383
(43) Date of publication of application: 17.04.2002
(73) Proprietor: Water Whole International, Inc., Norman, OK 73070-1263 (US)
(72) Inventor: SCHULHOFF, Jeffrey, Oklahoma City, OK 73162 (US); SCHALL, Christian, D-41334 Nettetal (DE)
(74) Representative: Prentice, Raymond Roy
(86) International application number: PCT/US2000/014939
(87) International publication number: WO 2000/072684

(56) References cited:
- EP-A- 0 379 256
- EP-A- 0 456 272
- EP-A- 0 656 417
- WO-A-96/17918
- WO-A-97/15649
- WO-A-97/39986
- DE-A- 2 520 988
- DE-A- 4 221 457
- US-A- 4 857 225
- US-A- 5 451 335
- US-A- 5 891 392
- US-A- 6 106 774

## Description

This invention relates generally to the cleaning of surfaces having deposits and more particularly, to the removal of particular types of deposits formed along these surfaces. Such surfaces are located along internal surfaces of drink water tanks, supply water wells, water filter systems, and distributor water lines.

Typically these surfaces are constantly being exposed to water and air. For example, the water level of a drink water tank rises and falls with demand. This constant change between water exposure and air exposure causes a biological film, such as algae and microorganisms, along with incrustations such as calcium, iron and manganese to form along these surfaces.

Cleaning these deposits from these surfaces has been the subject of a number of prior art compositions. U.S. Patent No. 4,199,469 issued to Walzer on April 22, 1980 discloses a composition and method for cleaning drink water tanks. This composition utilises a group of acids (ascorbic, formic, phosphoric, citric and hydrochloric).

Once the surfaces have been cleaned by a cleaning composition, a disinfectant is normally applied. This additional process adds time and resources to the overall cleaning process. Given the number of constituents the composition is complicated and confusing. Therefore, there exists a need for a more simple composition in order to clean surfaces having deposits contained thereon.

Cleaning compositions which include disinfectants have previously been proposed in DE-A-2520988 and US-A-5891392. However, both of these prior specifications are for industrial purposes and utilize very strong acids. The disclosed compositions are not intended for use with drink water tank applications because they create an acidic pH composition that would not be suitable for drinking water applications. The present invention aims to provide a composition and method for cleaning surfaces that avoids the problems mentioned above and which provides improvements over the existing compositions.

Accordingly, one aspect of the present invention provides a composition for cleaning surfaces having deposits comprising a cleaning solution portion and a disinfectant portion, in which the cleaning solution portion is selected from a mixture of sulfamic acid, citric acid, glycolic acid, phosphoric acid, hydrochloric acid, isopropanol, dyes and water, characterised in that the cleaning solution portion has the following volume percentages: sulfamic acid (0.1-2.2%), citric acid (0.2-10.0%), glycolic acid (0.1-14.6%), phosphoric acid (0.5-20%), isopropanol (0.1-1.0%), dyes (up to 009%) and water as the balance.

In a preferred composition, the cleaning solution portion has the following in a range by volume: phosphoric acid (5.0 - 20%), isopropanol (0.1-1.0%) and water as the balance.

In another preferred composition, the cleaning solution portion comprises an aqueous solution of amidosulfamic acid between 6.00 and 15.00 weight percent; nitrilotriacetic acid 0.10 and 20.00 weight percent; isopopanol between 0.10 and 0.50 weight percent; and water as the balance. Preferably, the cleaning solution components are in the relative weight percent proportions: 10.00 per cent amidosulfamic acid; 0.15 percent nitrilotriacetic acid; about 0.230 percent isopropanol; and water as the balance.

The cleaning solution portion is desirably in a range of 90-99.9% by volume and the disinfectant portion is in a range of 0.1-1.0% by volume.

The cleaning solution portion generally includes hydrochloric acid and/or phosphoric acid in combination with dyes and water. The disinfectant is a standard disinfectant, such as peracidic acid or hydrogen peroxide.

Another aspect of the present invention provides a method of cleaning surfaces having deposits thereon, the method comprising the steps of applying any one of the above-described compositions to said surfaces and rinsing the surfaces with water.

The composition is applied to the surfaces having deposits of biological film, such as algae and micro-organisms and/or incrustations such as calcium, iron and manganese thereon. The solution loosens the adhesion between the deposits and the surface at which time the composition and deposits are rinsed away. Further, the biological film is reliably eliminated.

The composition is preferably applied to surfaces by pressurised spraying, preferably low pressurised spraying.

The preferred embodiment of the present invention is directed toward the cleaning of surfaces specifically for the removal of deposits which have formed thereon and providing a disinfectant in combination.

With respect to the cleaning solution portion, one basic formula is:

| **Ingredients** | **Weight Percentage (Preferred)** |
|---|---|
| Sulfamic Acid | 2.0% |
| Dyes | 0.009% |
| Water | Balance |

Additional formulas include:

| **Ingredients** | **Weight Percentage (Range)** |
|---|---|
| Phosphoric Acid | 5.0% - 20.0% |
| Isopropanol | 0.1 - 1.0% |
| Water | Balance |

| **Ingredients** | **Range (Weight Percentage** | |
|---|---|---|
| | **Broad** | **Preferred** |
| Amidosulfamic Acid | 4 - 30% | 10% |
| Nitrilotriacetic Acid | 0.1% - 0.2% | 0.15% |
| Isopropanol | 0.1% - 0.5% | 0.3% |
| Water | Balance | Balance |

A disinfectant portion is added to cleaning solution in order to provide a disinfecting capacity. The disinfectant portion is a standard disinfectant, such as hydrogen peroxide or peracidic acid. The range of the disinfectant portion is 0.1 to 10.0% by volume of the entire composition.

The composition of the preferred embodiment of the present invention is applied to the surface where deposits have formed. These deposits comprise the combination of a biological film, such as algae and microorganisms, with sediments, such as calcium, iron and/or manganese. The composition of the preferred embodiment loosens the adhesive bond between the deposits and the wall surface allowing for the removal thereof. The composition is then rinsed away along with the loosened deposits as well as any remaining biological film.

## Claims

1. A composition for cleaning surfaces having deposits comprising a cleaning solution portion and a disinfectant portion, in which the cleaning solution portion is selected from a mixture of sulfamic acid, citric acid, glycolic acid, phosphoric acid, isopropanol, dyes and water, **characterised in that** the cleaning solution portion has the following volume percentages: sulfamic acid (0.1-2.2%), citric acid (0.2-10.0%), glycolic acid (0.1-14.6%), phosphoric acid (0.5-20%), isopropanol (0.1-1.0%), dyes (0.009%) and water as the balance.

2. A composition according to claim 1, **characterized in that** the cleaning solution portion has the following in a range by volume: phosphoric acid (5.0 to 20.0%), isopropanol (0.1 - 1.0%) and water as the balance.

3. A composition according to claim 1, **characterized in that** the cleaning solution portion comprises an aqueous solution of amidosulfamic acid between 6.00 and 15.00 weight percent; nitrilotriacetic acid between 0.10 and 20.00 weight percent; isopropanol between 0.10 and 0.50 weight percent; and water as the balance.

4. A composition according to claim 3, **characterised in that** the cleaning solution components are in the relative weight percent proportions: 10.00 per cent amidosulfamic acid; 0.15 percent nitrilotriacetic acid; 0.230 percent isopropanol; and water as the balance.

5. A composition according to any one of the preceding claims, **characterised in that** the cleaning solution portion is in a range of 90 to 99.9% by volume and the disinfectant portion is in a range of 0.1% to 10.0% by volume.

6. A composition according to any one of the preceding claims, **characterized in that** the disinfectant portion is defined as peracidic acid.

7. A composition according to any one of claims 1 to 6, **characterized in that** the disinfectant portion is defined as hydrogen peroxide.

8. A method of cleaning surfaces having deposits thereon, **characterised in that** the method comprises the steps of applying the composition claimed in any one of the preceding claims to said surfaces; and rinsing the surfaces with water.

9. A method according to claim 8, **characterized in that** the composition is applied to said surfaces by pressurized spraying, preferably low pressurized spraying.

## Patentansprüche

1. Zusammensetzung zur Reinigung von Flächen, die Ablagerungen aufweisen, umfassend einen Reinigungslösungsanteil und einen Desinfektionsmittelanteil, wobei der Reinigungslösungsanteil aus einer Mischung von Sulfamidsäure, Zitronensäure, Glykolsäure, Phosphorsäure, Isopropanol, Farbstoffen und Wasser ausgewählt ist, **dadurch gekennzeichnet, dass** der Reinigungslösungsanteil folgende Volumenprozentsätze aufweist: Sulfamidsäure (0,1 - 2,2 %), Zitronensäure (0,2 - 10,0 %), Glykolsäure (0,1 - 14,6 %), Phosphorsäure (0,5 - 20 %), Isopropanol (0,1 - 1,0 %), Farbstoffe (0,009 %) und Wasser als restlichem Anteil.

2. Zusammensetzung nach Anspruch 1, **dadurch gekennzeichnet, dass** der Reinigungslösungsanteil Folgende im Volumenbereich aufweist: Phosphorsäure (5,0 bis 20,0 %), Isopropanol (0,1 - 1,0 %) und Wasser als restlichem Anteil.

3. Zusammensetzung nach Anspruch 1, **dadurch gekennzeichnet, dass** der Reinigungslösungsanteil eine wässrige Lösung von Amidosulfamidsäure zwischen 6,00 und 15,00 Gewichtsprozent, Nitriltriessigsäure zwischen 0,10 und 20,00 Gewichtsprozent, Isopropanol zwischen 0,10 und 0,50 Gewichtsprozent und Wasser als restlichem Anteil umfasst.

4. Zusammensetzung nach Anspruch 3, **dadurch gekennzeichnet, dass** die Reinigungslösungsbestandteile in den relativen Gewichtsprozentanteilen: 10,00 Prozent Amidosulfamidsäure, 0,15 Prozent Nitriltriessigsäure, 0,230 Prozent Isopropanol und Wasser als restlichem Anteil vorliegen.

5. Zusammensetzung nach einem der vorhergehenden Ansprüche, **dadurch gekennzeichnet, dass** der Reinigungslösungsanteil im Bereich von 90 bis 99,9 Volumenprozent und der Desinfektionsmittelanteil im Bereich von 0,1 Volumen-% bis 10,0 Volumen-% vorliegt.

6. Zusammensetzung nach einem der vorhergehenden Ansprüche, **dadurch gekennzeichnet, dass** der Desinfektionsmittelanteil als Peressigsäure definiert ist.

7. Zusammensetzung nach einem der Ansprüche 1 bis 6, **dadurch gekennzeichnet, dass** der Desinfektionsmittelanteil als Wasserstoffperoxid definiert ist.

8. Verfahren zur Reinigung von Oberflächen, auf denen sich Ablagerungen befinden, **dadurch gekennzeichnet, dass** das Verfahren die Schritte des Aufbringens der in einem der vorhergehenden Ansprüche beanspruchten Zusammensetzung auf die Flächen und das Abspülen der Flächen mit Wasser umfasst.

9. Verfahren nach Anspruch 8, **dadurch gekennzeichnet, dass** die Zusammensetzung durch Spritzen unter Druck, bevorzugt Spritzen unter geringem Druck auf die Flächen aufgebracht wird.

## Revendications

1. Composition de nettoyage de surfaces porteuses de dépôts, comprenant une partie de solution nettoyante et une partie de désinfectant, la partie de solution nettoyante étant choisie parmi un mélange d'acide sulfamique, d'acide citrique, d'acide glycolique, d'acide phosphorique, d'isopropanol, de colorants ou de teintures et d'eau, **caractérisée en ce que** la partie de solution nettoyante présente les pourcentages volumiques suivants : acide sulfamique (0,1 à 2,2 %), acide citrique (0,2 à 10,0 %), acide glycolique (0,1 à 14,6 %), acide phosphorique (0,5 à 20 %), isopropanol (0,1 à 1,0 %), colorants ou teintures (0,009 %), le reste étant de l'eau.

2. Composition selon la revendication 1, **caractérisée en ce que** la partie solution nettoyante présente les plages suivantes en pourcentages volumiques : acide phosphorique (5,0 à 20 %), isopropanol (0,1 à 1,0 %), le reste étant de l'eau.

3. Composition selon la revendication 1, **caractérisée en ce que** la partie de solution nettoyante comprend une solution aqueuse d'acide amidosulfamique entre 6,00 et 15,00 % en poids ; de l'acide nitrilotriacétique entre 0,10 et 20,00 % en poids ; de l'isopropanol entre 0,10 et 0,50 % en poids, le reste étant de l'eau.

4. Composition selon la revendication 3, **caractérisée en ce que** les composants de la solution nettoyante sont présents dans les proportions relatives suivantes (pourcentages en poids) : 10 % d'acide amidosulfamique, 0,15 % d'acide nitrilotriacétique, 0,230 % d'isopropanol, le reste étant de l'eau.

5. Composition selon l'une quelconque des revendications précédentes, **caractérisée en ce que** la partie de solution nettoyante est présente dans la plage de 90 à 99,9 % en volume et la partie de désinfectant dans la plage de 0,1 à 10,0 % en volume.

6. Composition selon l'une quelconque des revendications précédentes, **caractérisée en ce que** la partie de désinfectant est définie comme étant un péracide.

7. Composition selon l'une quelconque des revendications 1 à 6, **caractérisée en ce que** la partie de désinfectant est définie comme étant du peroxyde d'hydrogène.

8. Procédé de nettoyage de surfaces porteuses de dépôts, **caractérisé en ce qu'**il comprend les étapes consistant à appliquer sur lesdites surfaces la composition revendiquée selon l'une quelconque des revendications précédentes, puis à rincer les surfaces à l'eau.

9. Procédé selon la revendication 8, **caractérisé en ce que** la composition est appliquée sur lesdites surfaces par pulvérisation sous pression, de préférence pulvérisation basse pression.
